# EUROPÄISCHE PATENTSCHRIFT

(11) **EP 1 004 276 B1**
(45) Veröffentlichungstag und Bekanntmachung des Hinweises auf die Patenterteilung: **05.02.2003**
(21) Anmeldenummer: 00101434.9
(22) Anmeldetag: 25.11.1997
(51) Int. Cl.: A61B 17/70, A61B 17/72, A61B 17/74

(54) **Prophylaxe-Implantat gegen Frakturen osteoporotisch befallener Knochensegmente**
Prophylactic implant for protecting bone segments affected by osteoporosis against fractures
Implant à usage prophylactique servant à mettre des segments osseux atteints d'ostéoporose à l'abri de fractures

(30) Priorität: 18.12.1996 DE 19652608
(43) Veröffentlichungstag der Anmeldung: 31.05.2000
(62) Teilanmeldung aus: 97951969.1
(73) Patentinhaber: ESKA Implants GmbH & Co., 23556 Lübeck (DE)
(72) Erfinder: Grundei, Hans, 23558 Lübeck (DE); Thomas, Wolfram, 00135 Roma (IT)
(74) Vertreter: Fuchs Mehler Weiss & Fritzsche

(56) Entgegenhaltungen:
- EP-A- 0 636 346
- WO-A-96/39974
- DE-A- 19 508 224
- DE-C- 4 208 247
- DE-U- 29 509 739
- US-A- 2 537 070

## Beschreibung

Die vorliegende Erfindung betrifft ein Prophylaxe-Implantat gegen Frakturen osteoporotisch befällener Knochensegmente.

Allein in Deutschland gibt es Millionen von Osteoporotikern, die also unter einer der häufigsten Erkrankungen des Bewegungsapparates leiden.

Bei der Osteoporose findet ein Verlust von Knochenmasse statt, der über den natürlichen altersbedingten Knochenabbau hinausgeht. Darüber hinaus verschlechtert sich die Qualität der Mikroarchitektur des Knochengewebes. Der Verlust an Knochenmasse, Knochenstruktur und Funktion führt dann zu klinischen Symptomen mit dauerhaften Beschwerden und Frakturen. Besonders weit verbreitet sind Schenkelhalsbrüche, Frakturen der Wirbelsäule und am Handgelenk.

Bei osteoporotischen Frakturen des Schenkelhalses kommt als Therapie heutzutage im wesentlichen nur die Totalresektion des Femurs und des natürlichen Acetabulums sowie die Implantation eines kompletten künstlichen Hüftgelenksystem in Betracht. Diese Systeme sind heute zwar ausgereift, der hierfür nötige Eingriff ist allerdings nach wie vor als ein schwerer Eingriff zu werten. Insbesondere kritisch ist aber darüber hinaus nach wie vor die Langzeitstabilität des Implantates im Patientenkörper. Einflüsse hierauf haben beispielsweise die Art des Implantates (zementfrei; zementiert), Alter und Konstitution des Patienten neben vielen anderen Parametern. Allein von daher wäre es wünschenswert, wenn die Totalresektion von Knochensegmenten so lang wie möglich auch bei Osteoporotikern hinausgezögert werden könnte oder gar völlig überflüssig werden würde.

Ansatzpunkte für eine Früherkennung des voraussichtlichen Traumas oder der Fraktur des betroffenen Knochensegmentes ergeben sich aus neuesten radiologischen Untersuchungen. So sind Zeichen der osteoporose-bedingten Veränderungen der Knochenstruktur radiologisch eindeutig feststellbar und interessanterweise ist der Zeitpunkt der voraussichtlichen Fraktur innerhalb eines Zeitraumes von bis zu einem Jahr mit erstaunlich hoher Genauigkeit vorhersagbar. Diese Erkenntnis bietet die Gelegenheit und die Chance, vor der Fraktur therapeutisch einzugreifen und die Osteoporose zum Stillstand zu bringen oder zurückzudrängen.

Ansatzweise ist versucht worden, das aufgezeigte Problem mit einer hohlen Hülse zu lösen, wie sie bekannt ist aus der US-A-2 537 070. Diese hohle Hülse wird in eine im Schenkelhals freigeräumte Bohrung gesetzt. Die Hülse verfügt über eine Anzahl von Durchbrechungen, durch welche hindurch das die Hülse nach der Implantation umgebende Knochenmaterial in das Innere der Hülse greifen kann und dort mit pulverisiertem Knochenmaterial in Kontakt treten kann. Schließlich soll das die Hülse umgebende Knochenmaterial sich mit dem pulverisierten Knochen in der Hülse verfestigen, da daß das Implantat langfristig als Armierungsimplantat wirken können soll.

Bei diesem bekannten Implantat wird es als nachteilig angesehen, daß die Kontaktfläche zwischen dem natürlichen Knochen und dem pulverisierten Knochenmaterial im Inneren der Hülse recht klein ist. Ist kein pulverisiertes Knochenmaterial im Inneren der Hülse vorgesehen, so lassen die Öffnungen bei dem bekannten Implantat ein nur sehr langsames Einsprossen von Knochenmaterial in das hohle Innere der Hülse zu. Hierdurch werden die Erfolge bei Einsatz des Implantats als Prophylaxe-Implantat geschmälert.

Vor diesem Hintergrund ist es die Aufgabe der vorliegenden Erfindung, ein verbessertes Prophylaxe-Implantat gegen Frakturen osteoporotisch befallener Knochensegmente anzugeben.

Gelöst wird die Aufgabe durch das im Anspruch 1 angegebene Prophylaxe-Implantat. Weitere vorteilhafte Ausführungsformen sind in den Unteransprüchen angegeben.

Demgemäß ist vorgesehen, daß das Implantat ein Hohlrohr aufweist, dessen Außenwand gebildet ist aus einer offenmaschigen, dreidimensionalen Raumnetzstruktur. Durch diese Raumnetzstruktur kann Knochenmaterial von außen in das Innere der Hülse wachsen. Es bedarf bei dem vorliegenden Vorschlag keinerlei zusätzlicher Bereitstellung eines Mechanismus oder einer besonderen Außenstruktur, um das Implantat an einem Herauswandern aus dem Knochensegment zu hindern, da nämlich die offenmaschige, dreidimensionale Raumnetzstruktur, die gleichzeitig die Hülsenwandung bildet, vom Knochen durch- und umwachsen wird und so die Rückhaltefunktion ausüben kann.

Das erfindungsgemäße Prophylaxe-Implantat kann noch dadurch vorteilhaft weitergebildet werden, daß das Hohlrohr eine Hülse bildet, welche von proximaler Seite her mit einem Verschlußstück verschließbar ist, wobei das Verschlußstück selbst ebenfalls aus einer offenmaschigen, dreidimensionalen Raumnetzstruktur besteht. Diese Ausführungsform gestattet es, das Hülseninnere optional mit einem heilungsfördernden Mittel (Hydroxylapatit, alpha-Tricalciumphosphat-Keramik, etc.) zu füllen, um den Einheilprozeß zu beschleunigen. Die Raumnetzstruktur des Verschlußstückes dient in diesem Fall ebenfalls wieder dem Hindurchwachsen von Knochenmaterial.

Dem erfindungsgemäßen Prophylaxe-Implantat liegt die Philosopie zugrunde, eine Fraktur des osteoporotisch befallenen Knochensegmentes dadurch hinauszuzögern oder zu verhindern, daß zunächst der osteoporotisch befallene Knochen ausgefräst wird und an die Stelle der krankhaften Spongiosa das erfindungsgemäße Implantat gesetzt wird, welches von gesundem nachwachsenden Knochenmaterial durchwachsen wird, wodurch schließlich die Stabilität des gesamten Knochens wiederhergestellt oder zumindest deutlich erhöht wird.

Gemäß einer Weiterentwicklung des Implantates ist vorgesehen, daß im Armierungskörper auf einem darin zentriert angeordneten magnetischen Kern eine elektrische Spule angeordnet ist, die von extern her anregbar ist.

Die vorliegende Erfindung macht sich die sogenannte magnetisch induzierte Elektrostimulation in der Orthopädie zunutze, wie sie ausführlich in der einschlägigen Fachliteratur beschrieben ist. Beispielsweise sei hingewiesen auf den Aufsatz "Magnetfeldtherapie und magnetisch induzierte Elektrostimulation in der Orthopädie", W. Kraus, DE-Z-Orthopäde (1984) 13:78 bis 92.

Die auf dem magnetischen Kern angeordnete Spule ist in dieser Konfiguration anzusehen als die Sekundärwicklung eines Transformators, dessen Primärwicklung von extern her an den Patienten gelegt wird. Ein niederfrequenter Wechselstrom in der Primärwicklung erzeugt eine ebenso niederfrequente Spannung an den Klemmen der Sekundärwicklung auf dem magnetischen Kern. In der Sekundärwicklung fließt dann über das Füllmaterial im Inneren des Armierungskörpers ein elektrischer Strom, welcher das Knochenwachstum in bekannter Weise stimuliert.

Als Resultat wird der Armierungskörper schneller von natürlicher Spongiosa durchwachsen, wodurch die Struktur und die Stabilität des befallenen Knochens deutlich schneller wiederhergestellt wird.

Gemäß einer vorteilhaften Weiterbildung kann vorgesehen sein, daß der magnetische Kern als Gewindespindel ausgebildet ist, welche dazu dient, die in diesem Falle aus Kunststoff bestehende zylindrische Hülse des Armierungskörpers durch Drehung der Gewindespindel zu stauchen, um so eine Wanderungsneigung des Implantates im Knochenkanal zu unterbinden.

Gemäß einer noch weiteren vorteilhaften Ausführungsform ist vorgesehen, daß das Kopfteil einen in das Innere des Armierungskörpers ragenden Fortsatz mit einer die zentrierte Lage des magnetischen Kerns sichernden Aufnahme aufweist. Das Kopfteil und der Fortsatz kann beispielsweise und bevorzugterweise aus einer geeigneten Keramik bestehen. Ist beispielsweise die Spitze des magnetischen Kerns kegelförmig ausgebildet, so ist die Aufnahme vorzugsweise komplementär hierzu in der Keramik vorgesehen.

Die Erfindung wird anhand eines Ausführungsbeispiels anhand der Zeichnungsfiguren näher erläutert. Hierbei zeigt:
- Figur 1: die Ansicht des Implantates
- Figur 2: die Ansicht des Implantates gemäß einer Weiterentwicklung, teilweise weggebrochen
- Figur 3: eine schematische Ansicht der Implantationslage des Implantates aus Fig. 2 im Femurschenkelhals, und
- Figur 4: eine schematische Ansicht der Implantationslage des Implantates in einem Wirbelkörper.

In den Figuren sind gleiche Teile jeweils mit denselben Bezugszeichen versehen.

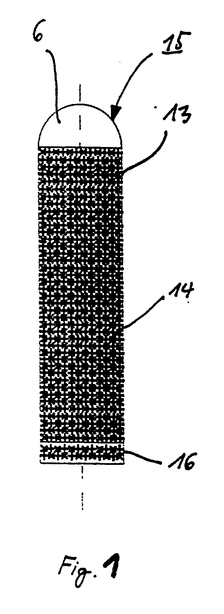

Fig. 1 zeigt ein Schenkelhalsimplantat, welches als Hohlrohr 13 ausgebildet ist. Die Außenwandung des Hohlrohres 13 wird gebildet aus einer offenmaschigen dreidimensionalen Raumnetzstruktur 14, durch welche Knochenmaterial hindurchwachsen soll. Distal abgeschlossen ist das eine Hülse bildende Hohlrohr 13 mit dem Kopfteil 6.

Proximal ist vorliegend ein Verschlußstück 16 angeordnet, durch welches die Hülse 15 verschlossen werden kann.

Es bedarf keinerlei Maßnahme, um das Implantat an einem Auswandern aus dem in den Schenkelhals einzubringenden Knochenkanal zu hindern, da nämlich die Raumnetzstruktur 14 selbst für die Lagestabilität sorgt.

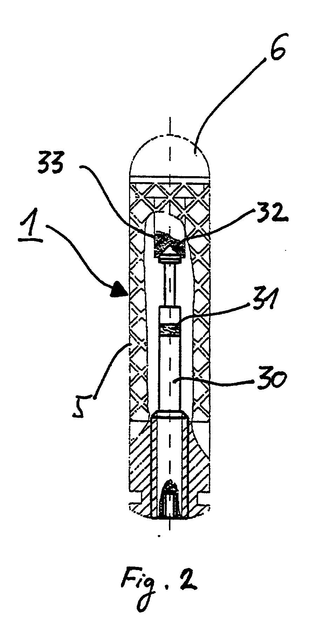

Wie in Figur 2 schematisch dargestellt, ist der Armierungskörper 1 eine im wesentlichen hohe zylindrische Hülse 5. Darüber hinaus ist im Inneren der Hülse 5 ein magnetischer Kern 30 zentriert angeordnet. Der magnetische Kern 30 trägt eine Spule 31, welche die Sekundärentwicklung eines Transformators ist, der gebildet ist aus der Spule 31 und einer extern an den Patientenkörper heranzuführenden Primärentwicklung oder Spule (nicht dargestellt), durch welche ein niederfrequenter Wechselstrom von ca. 0,2 bis 20 Hz geleitet wird, um die Therapie einzuleiten. Dieser Wechselstrom im Primärkreis erzeugt einen Strom im Sekundärkreis, nämlich in der Spule 31 und in dem zwischen beiden Spulenklemmen befindlichen Füllmaterial im Inneren der Hülse 5.

Das Kopfteil 6 der Hülse 5 weist einen in das Innere des Armierungskörpers 1 ragenden Fortsatz 33 auf, der an seinem stirnseitigen Ende eine komplementär zu der Spitze des magnetischen Kerns 30 ausgebildete Aufnahme 32 aufweist. Das Kopfteil 6 besteht vorzugsweise aus einer körperverträglichen Keramik.

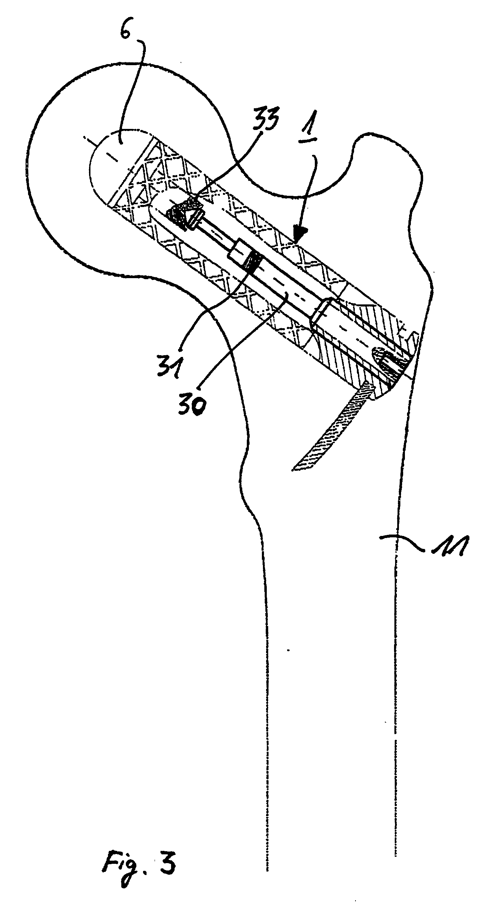

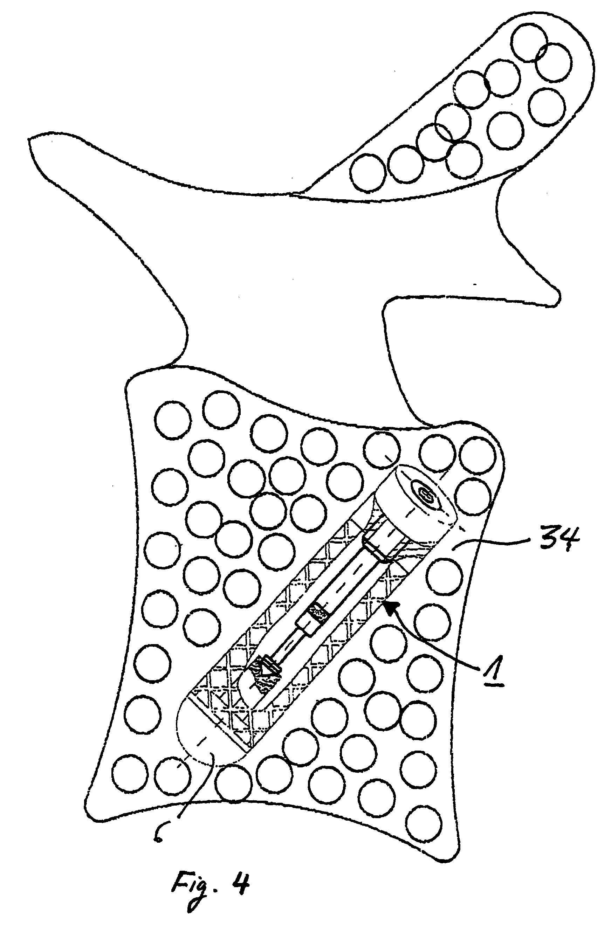

Figur 3 veranschaulicht zur Vervollständigung des Bildes die Implantationslage des Implantates im Schenkelhals des Femurs 11. Eine ähnliche Ausführungsform der Armierungshülse 1 kann vorgesehen sein zum Einsatz in einem Wirbelkörper 34, wie dies in Figur 4 dargestellt ist. Freilich sind die Dimensionen andere als bei dem Implantat, welches in den Schenkelhals eines Femurs eingesetzt werden soll.

## Patentansprüche

1. Prophylaxe-Implantat gegen Frakturen osteoporotisch befallener Knochensegmente, insbesondere des Schenkelhalses, der Wirbelsäule und des Handgelenkes, aufweisend ein Hohlrohr (13), **dadurch gekennzeichnet, dass** die Außenwandung des Hohlrohrs (13) gebildet ist aus einer offenmaschigen, dreidimensionalen Raumnetzstruktur (14).

2. Implantat nach Anspruch 1, bei dem das Hohlrohr eine Hülse (15) bildet, welches von proximaler Seite her mit einem Verschlußstück (16) verschließbar ist, wobei das Verschlußstück (16) ebenfalls aus einer offenmaschigen, dreidimensionalen Raumnetzstruktur besteht.

3. Implantat nach Anspruch 1 oder 2, bei dem im Armierungskörper (1) auf einem darin zentriert angeordneten magnetischen Kern (30) eine elektrische Spule (31) angeordnet ist, die von extern anregbar ist.

4. Implantat nach Anspruch 3, bei dem der magnetische Kern (30) als Gewindespindel ausgebildet ist.

5. Implantat nach Anspruch 3 oder 4, bei dem das Kopfteil (6) des Armierungskörpers einen in das Innere des Armierungskörpers (1) ragenden Fortsatz (33) mit einer die zentrierte Lage des magnetischen Kerns (30) sichernden Aufnahme (32) aufweist.

## Claims

1. Prophylactic implant against fractures of bone segments affected by osteoporosis, in particular the neck of the femur, the spinal column and the wrist joint, having a hollow tube (13), **characterised in that** the outer wall of the hollow tube (13) is formed from an open-meshed, three-dimensional spatial network structure (14).

2. Implant according to claim 1, in which the hollow tube forms a sleeve (15) which can be closed from the proximal side using a closure piece (16), wherein the closure piece (16) likewise consists of an open-meshed, three-dimensional spatial network structure.

3. Implant according to claim 1 or 2, in which an electric coil (31), which can be stimulated externally, is arranged in the reinforcing body (1) on a magnetic core (30) arranged to be centred therein.

4. Implant according to claim 3, in which the magnetic core (30) is designed as a threaded spindle.

5. Implant according to claim 3 or 4, in which the head part (6) of the reinforcing body has an extension (33) projecting into the interior of the reinforcing body (1) with a recess (32) ensuring the centred position of the magnetic core (30).

## Revendications

1. Implant prophylactique contre les fractures de segments osseux atteints d'ostéoporose, en particulier du col du fémur, des vertèbres et de l'articulation de la main, présentant un tube creux (13), **caractérisé en ce que** le revêtement externe du tube creux (13) est formé en une structure en réseau tridimensionnelle à mailles ouvertes (14).

2. Implant selon la revendication 1 dans lequel le tube creux forme un fourreau (15), qui peut être fermé en son extrémité proximale avec un bouchon (16), le bouchon (16) étant également fabriqué en une structure en réseau tridimensionnelle à mailles ouvertes.

3. Implant selon la revendication 1 ou 2 dans lequel dans le corps armé (1), sur un noyau magnétique (30) centré, est placée une bobine électrique (31), qui peut être activée de l'extérieur.

4. Implant selon la revendication 3 dans lequel le noyau magnétique (30) est formé comme une tige filetée.

5. Implant selon la revendication 3 ou 4 dans lequel la tête (6) du corps armé présente une apophyse (33) se dressant à l'intérieur du corps armé (1) présentant elle-même un logement (32) assurant la position centrée du noyau magnétique (30).
